# EUROPEAN PATENT SPECIFICATION

(11) **EP 4 454 540 B1**
(45) Date of publication and mention of the grant of the patent: **12.11.2025**
(21) Application number: 24172169.5
(22) Date of filing: 24.04.2024
(51) Int. Cl.: A61B 1/015, A61B 1/12, A61B 1/313, A61B 17/34

(54) **ACCESS ASSEMBLY FOR SURGICAL INSTRUMENT**
ZUGANGSANORDNUNG FÜR EIN CHIRURGISCHES INSTRUMENT
ENSEMBLE D'ACCÈS POUR INSTRUMENT CHIRURGICAL

(30) Priority: 26.04.2023 CN 202321009589 U
(43) Date of publication of application: 30.10.2024
(73) Proprietor: NINGBO HITCM MEDICAL DEVICES CO., LTD., Ningbo, Zhejiang 315000 (CN)
(72) Inventor: DING, Weijiang, Ningbo, Zhejiang 315000 (CN); YUAN, Peng, Ningbo, Zhejiang 315000 (CN); CAO, Xindong, Ningbo, Zhejiang 315000 (CN)
(74) Representative: Viering, Jentschura & Partner mbB Patent- und Rechtsanwälte

(56) References cited:
- CN-A- 113 288 019
- CN-U- 216 317 505

## Description

### TECHNICAL FIELD

The present disclosure relates to an access assembly, in particular to an access assembly for a surgical instrument.

### BACKGROUND

During the surgical procedure, a surgeon usually needs to diagnose and/or treat relevant organs in a patient's body by means of surgical instruments (such as forceps, graspers, cutters, applicators, etc.), which must be inserted into the patient's body by means of a cannula. However, operatively executive parts of the surgical instruments received into the patient's body are easily adhered with condensates, tissues, bloods and other body fluids in the body cavity during the diagnosis and/or treatment, which adversely affects or impairs the effectiveness of diagnosis and/or treatment. Therefore, it is very important to keep the relevant surgical instruments, especially the lens of the endoscope, clean during the surgery. Conventionally, the surgeon (e.g., an endoscopist) withdraws the endoscope from an incision on the patient's body via an access assembly, cleans the lens with pre-prepared physiological saline at a body temperature, then wipes the endoscope with a disinfectant such as iodophor and reinserts the endoscope into the incision on the patient's body via the access assembly. During the surgical procedure, the time required to clean the lens may increase the total time of the surgery and the amount of time the patient needs to remain anesthetized; furthermore, since the endoscope is repeatedly withdrawn from and inserted into the incision on the patient's body, this may result in increased risk of infection and also prolong the recovery time. Therefore, there is a need in the art for an improved access assembly that can easily and quickly clean the lens of an endoscope during a surgery without introducing additional irrigation liquid or dirt into the body cavity of a patient.

An access assembly for an endoscope of the type according to the present disclosure is known, but there is no gas-liquid mixture supply device in the access assembly, so the suction speed under negative pressure is slow, and the shape of the saline channel makes it difficult to process, and the gradual channel is arranged on the cannula (which is not arranged on a distal sealing device).

An access assembly for an endoscope is known to have a three-way tube, which is T-shaped and installed on a proximal seal. Although the three-way tube can realize gas-liquid mixing, the whole length of the puncture device is increased, which adversely affects the surgical operation of a surgeon. **In** other words, one of disadvantages of such access assembly is that the longer length of the whole access assembly affects the surgical operation. Another disadvantage is that the three-way tube is installed on the proximal sealing device by being sleeved outside a first fluid supply inlet of the access assembly. Since the three-way tube is not integrally fixed with the access assembly, it is easy to fall off the access assembly, which hinders the access assembly to be reliably cleaned.

**In** CN113288019A, an access assembly for receiving an endoscope is disclosed, comprising: a first cannula component having a tubular inner wall, the distal end of which is operatively inserted into a body cavity of a patient from an incision in the body of the patient; a second sleeve part is arranged in the first sleeve part and is provided with an elongated body, and the elongated body comprises a tubular outer wall which extends in the direction of the longitudinal axis of the endoscope and is matched with the tubular inner wall of the first sleeve part in a fluid sealing manner, and a tube cavity used for receiving the endoscope; and a vacuum sealing component is arranged at the far end of the long and narrow body and has an open state, a one-way valve state and a closed state. The vacuum seal member is in a one-way valve state when the distal end of the endoscope is retracted into the lumen during lens cleaning and lens drying. A method of intraoperatively cleaning a lens of an endoscope using the access assembly is also disclosed.

### SUMMARY

The technical problem to be solved by the present disclosure is to provide an improved access assembly for surgical instruments. The access assembly with a simplified structure can be reliably cleaned and especially allows for a reliable cleaning effect.

This technical problem is solved by an access assembly for a surgical instrument according to the independent claim of the present disclosure.

The developed embodiments are defined in the corresponding dependent claims.

### BRIEF DESCRIPTION OF DRAWINGS

The above-mentioned characteristics, features and advantages of the present disclosure and the way to realize them can be more clearly understood by combining with the following description of non-limiting embodiments set forth in more detail with reference to the accompanying drawings. As shown in the accompanying drawings:
Fig. 1 is a side cross-sectional view of a non-limiting embodiment of an access assembly of an accessed surgical instrument according to the present disclosure;
Fig. 2 is a side view of the access assembly in Fig. 1.

### DETAILED DESCRIPTION

The following embodiments are preferred implementations of the present disclosure. In these embodiments, the individual components of the described embodiments form individual features of the present disclosure which are considered separately and independently from each other. These individual components also expand the present disclosure independently from each other, and hence can also form a part of the present disclosure separately, or in other forms different from the illustrated combination(s). In addition, the described implementations can also be supplemented by other features of the present disclosure already described.

Before specifically explaining the various aspects of the access assembly and various features thereof, it should be noted that the various aspects disclosed herein are not limited in their application or usage to the details of construction and arrangement of the components and parts illustrated in the drawings and description. Rather, the disclosed device may be located or combined into other devices, variations and modifications thereof, and may be practiced or performed in various ways. Accordingly, aspects of features of the access assembly disclosed herein are illustrative in nature and are not meant to limit their scope or application. Furthermore, unless otherwise specified, the terms and expressions used herein have been chosen for the convenience of the reader to understand various aspects of the characteristics of the access assembly and are not intended to limit the scope thereof. Furthermore, it should be understood that any one or more components of the access assembly, expressions thereof, and/or examples thereof may be combined with any one or more other components, expressions thereof, and/or examples thereof without limitative.

An access assembly for a surgical instrument is described in details with reference to the accompanying drawings, in which the same reference numerals refer to the same or corresponding elements in each of the above views. As used herein, the technical term "surgical instrument" refers to any surgical instrument that can be received into a subject's body through an access assembly such as a puncture device, and specifically refers to an endoscope in the non-limiting embodiments of the present disclosure; the technical term "tube member" refers to a cannula of the puncture device; the terms "distal" and "distal side" refer to a direction (for example, the downward direction as illustrated in the drawings) in which the surgical instrument, a portion or a component of the access assembly are farther away from an operator (for example, a surgeon); further, the term "distal direction" refers to a direction along which the surgical instrument, especially the endoscope, is inserted, and the term "distal end" refers to an end away from the surgical instrument, especially the endoscope, relative to the operator (e.g., the surgeon); on the contrary, the term "proximal" refers to a direction (for example, the upward direction as illustrated in the drawings) in which the endoscope, a portion or a component of the access assembly are closer to the operator, further, the term "proximal direction" refers to a direction along which the surgical instrument, especially the endoscope, is withdrawn, and the term "proximal end" refers to an end closer to the surgical instrument, especially the endoscope, relative to the operator (e.g., the surgeon). Additionally, in general conception, the term "endoscope" can be used interchangeably with any other equipment such as laparoscope, arthroscope, gastrointestinal endoscope, laryngo-bronchial endoscope, etc. for observing the body cavity of a patient through an incision or a cannula with small diameter. As used herein, the term "fluid" generally refers to a substance with fluidity, including but not limited to liquid (such as pure liquid, solution, colloid, suspending liquid, turbid liquid), gas, gas-liquid two-phase fluid mixture, plasma, fluidized solid particle, etc.

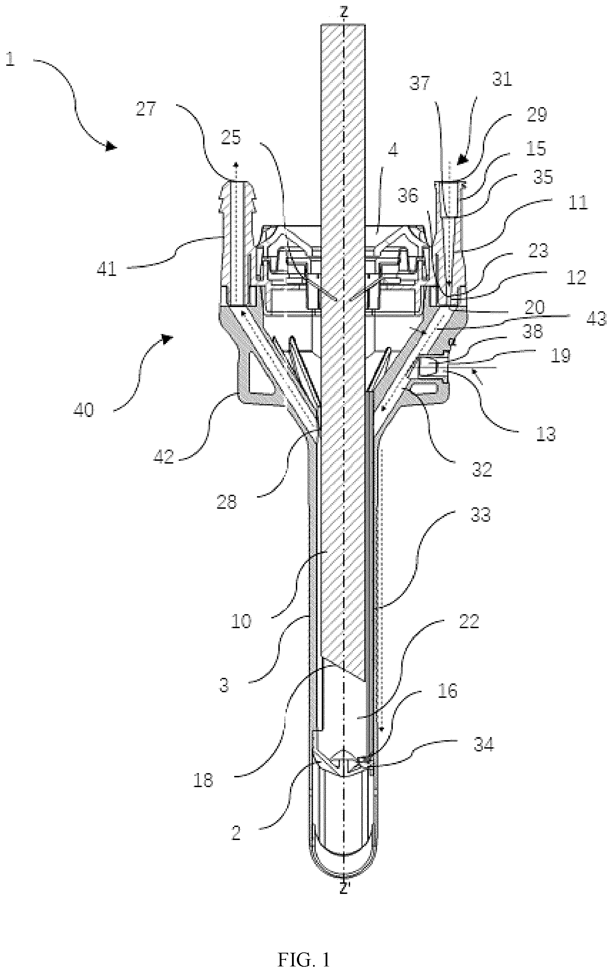

Fig. 1 is a side cross-sectional view of a non-limiting embodiment of an access assembly 1 according to the present disclosure in which a surgical instrument 10 has been received. The access assembly 1 includes a housing member 40, a tube member 3, a proximal sealing device 25 and a distal sealing device 2.

The tube member 3 is configured to receive the surgical instrument 10 and is structured to extend along a longitudinal axis ZZ' of the access assembly 1. The housing member 40 is located at a proximal side of the access assembly 1 and includes a longitudinal channel for receiving the surgical instrument. The tube member 3 is connected at a distal end of the housing member. The housing member 40 and the tube member 3 together constitute an entire longitudinal channel for receiving the surgical instrument 10. In an embodiment, the housing member 40 and the tube member 3 are integrally formed or detachably assembled with each other. The housing member 40 includes a shell component 4 surrounding an outer side of the longitudinal channel, and the shell component 4 at least includes a conical portion 42 and a cylindrical portion 41 connected with each other. The conical portion 42 and the cylindrical portion 41 are integrally formed or detachably assembled with each other.

In this embodiment, the shell component 4 has a proximal sealing device25 and is sealed with an outer wall of the surgical instrument 10 through the proximal sealing device 25. The proximal sealing device 25 abuts against the outer wall of the surgical instrument 10 to seal the outer wall when the surgical instrument 10 passes through the longitudinal channel of the housing member 40.

The distal sealing device 2 is designed as a W-shaped sealing element in this embodiment and is configured to be connected with a distal end of the tube member 3 in a sealed manner. The sealing effect can be achieved unidirectionally in such a manner that the distal sealing device 2 is installed at a distal end position of an inner surface of a cylindrical portion of the tube member 3, and an inner diameter of the inner surface of the cylindrical portion of the tube member 3 is equivalent to (for example, substantially equal to or slightly smaller than) an outer diameter of an outer wall of a tubular housing of the distal sealing device 2; in this way, a good sealing effect is achieved between the distal sealing device 2 and the tube member 3. The distal sealing device 2 may allow fluid to pass from the outside (for example, the body cavity of a patient) of the access assembly 1 to enter a cavity 22 of the access assembly 1 under a pressure difference in a direction from the cavity 22 of the access assembly 1 pointing to the outside of the access assembly 1, but does not allow fluid to leave the cavity 22 of the access assembly 1 to the outside (for example, the body cavity of the patient) of the access assembly 1 under a pressure difference in a direction from the outside of the access assembly 1 pointing to the cavity 22 of the access assembly 1.

A vacuum suction device (not shown) is configured to provide a negative pressure to the access assembly 1, and the access assembly has at least one vacuum suction channel (also referred to as a discharge passage), and the vacuum suction device is connected to the vacuum suction channel. The vacuum suction device also includes a vacuum suction interface 27 (also referred to as an outlet of the discharge passage) connected to a vacuum suction source (for example, a vacuum pump, a vacuum source used as a hospital infrastructure, etc.), a vacuum suction port 28 (also referred to as an inlet of a discharge passage), and a vacuum suction channel (also referred to as a discharge passage) that fluidly communicates the vacuum suction interface 27 with the vacuum suction port 28. The vacuum suction interface 27 is directly connected with the vacuum suction source, and the vacuum suction port 28 is opened into the cavity 22 of the access assembly to suck out the cleaning fluid and the dirt inside the cavity 22. The cleaning fluid and the dirt within the cavity 22 of the access assembly 1 can leave the access assembly 1 through the vacuum suction port 28 along the vacuum suction channel from the vacuum suction interface 27, by using the vacuum suction device.

Although the vacuum suction port 28 shown in Fig. 1 is located at a relatively proximal position in the tube member 3, the vacuum suction port 28 according to the present disclosure is not limited to this and may be provided at any suitable position in the tube member 3. It should be understood that during a surgery performed by using the access assembly 1 and the surgical instrument 10, depending on different working angles required by the surgical instrument 10 (for example, an upright orientation, an inverted orientation, a horizontal orientation, an obliquely downward orientation, an obliquely upward orientation, etc.), the access assembly 1 is also arranged at an angle as required correspondingly. Accordingly, the cleaning fluid and the dirt may be accumulated at a proximal or distal side within the cavity 22 of the access assembly due to gravity. In this case, the vacuum suction port may be correspondingly arranged at a proximal or distal side within the tube member 3 to facilitate the effect of sucking fluid and dirt out of the cavity 22.

The inner wall of the tube member 3, the distal surface of the proximal sealing device 25 and the proximal surface of the distal sealing device collectively define the cavity 22 of the access assembly 1 to form an interior space which is generally isolated from the external air at the proximal side of the access assembly 1 and the body cavity of the patient at the distal side of the access assembly 1. In some embodiments, the distal sealing device 2 may be configured such that when the surgical instrument 10 passes through the distal sealing device 2, a valve of the distal sealing device 2 opens and abuts against the surgical instrument 10 to realize sealing. In this way, for example, when the surgical instrument 10 is used for operation, especially when an endoscope is used for observing the surgical field, the body cavity of the patient maintains isolated from the cavity 22 of the access assembly 1.

In this embodiment, a fluid supply channel includes a first fluid supply channel 31 for inputting a first fluid, a mixing component, a third supply channel section 33 and a fourth supply channel section 34, sequentially, from a proximal end to a distal end of the fluid supply channel. The first fluid supply channel 31 extends from the proximal end to the distal end of the housing member 40 and is arranged in the cylindrical portion 41 of the housing member 40. The mixing component is arranged at the distal end of the housing member 40 and is in fluid communication with the first fluid supply channel 31. The fluid supply channel is configured to provide mixed fluid (gas-liquid mixed fluid in this embodiment) to the access assembly 1. The fluid supply channel can mix liquid such as physiological saline with cleaning gas, and can discharge the gas-liquid mixed fluid into the cavity 22 of the access assembly 1 to clean a distal end part 18 of the surgical instrument 10, which is preferably a lens of the endoscope. The first fluid supply channel 31 may be arranged in the cylindrical portion 41 of the shell component 4 and be configured to input a first fluid, preferably liquid, from the outside. A second channel of the mixing component (an embodiment in which a third channel is not provided) is located downstream of the first fluid supply channel 31, or a third channel of the mixing component (an embodiment in which the third channel is provided) is located downstream of the first fluid supply channel 31 and is in fluid communication with the first fluid supply channel 31. The mixing component may be arranged in the conical portion 42 of the housing member. The third supply channel section 33 is located downstream of the mixing component and in fluid communication with the mixing component. The fourth supply channel section 34 is a fluid channel arranged on the distal sealing device, and is located downstream of the third supply channel section 33 and in fluid communication with the third supply channel section 33.

The first fluid supply channel 31 has an inlet 29 for inputting the first fluid into the access assembly from the outside, and an outlet for outputting the first fluid to a second passage 32 (described later) of the mixing component. Furthermore, the first fluid supply channel 31 includes three channel sections, namely, a first channel section 11 whose cross-sectional area of flow is varied, an optional second channel section 12 whose cross-sectional area of flow is constant, and an optional third channel section 15 whose cross-sectional area of flow is basically constant. The first channel section 11 has an inlet 35 and an outlet 23, the second channel section 12 has an inlet 36 and an outlet 20, and the third channel section 15 has an inlet 29 (in this embodiment, it's the inlet 29 of the first fluid supply channel 31) and an outlet 37. The outlet 37 of the third channel section is in fluid communication with the inlet 35 of the first channel section 11, and the outlet 23 of the first channel section is in fluid communication with the inlet 36 of the second channel section. The first fluid supply channel 31 has a first channel section 11 with a varied cross-sectional area of flow and an optional second channel section 12 with a constant cross-sectional area of flow. The first channel section 11 with varied cross-sectional area of flow has a cross-sectional area of flow that decreases in the direction of fluid flow; the first fluid supply channel 31 is configured such that its cross-sectional area of flow decreases in a stepwise manner along the direction of fluid flow; alternatively, the first fluid supply channel 31 may also be configured such that its cross-sectional area of flow continuously decreases along the direction of fluid flow. The first channel section 11 is in fluid communication with a third passage 43 (described later) of the mixing component via the second channel section 12; the second channel section 12 has a constant cross-sectional area of flow in the direction of fluid flow, which is equal to the cross-sectional area of flow of the first channel section 11 with a varied cross-sectional area of flow at the outlet 23 of the first channel section 11.

The outlet 23 of the first channel section 11 with a varied cross-sectional area of flow limits the flow velocity of the liquid from the inlet 29 of the first fluid supply channel 31, in order to facilitate the effect of mixing the liquid with the gas into gas-liquid two-phase fluid. Since the cross-sectional area of the first fluid supply channel 31 at its inlet 29 is greater than that at its outlet 20, the flow rate of the liquid passing through the first fluid supply channel 31 is reduced, thereby lowering the demand for the supply pressure of the liquid source. Without being limited by any theory, a tapered cross-sectional area of flow gradually increases the flow velocity of the fluid in the first channel section 11 with a varied cross-sectional area of flow. According to Bernoulli's principle, this causes the static pressure of the fluid at the outlet 20 of the first fluid supply channel 31 to be decreased, so that it is easier to mix the first fluid with the second fluid, especially gas, from a second fluid supply channel 13 (also referred to as a first passage 13 of the mixing component, which will be described later) into a uniform two-phase fluid mixture, especially a gas-liquid two-phase mixture. As an example, the outlet 23 of the first channel section 11 with varied cross-sectional area of flow may be configured to exert respective resistances to the liquid, gas/cleaning agent and fluid mixture, thereby facilitating uniform mixing of the liquid and gas/cleaning agent.

In another embodiment, the first fluid is, for example, purified water, physiological saline, etc., while the third fluid is a cleaning agent, such as surfactant, and the first fluid and the third fluid are mixed into a uniform, cleaning agent-water solution or cleaning agent-physiological saline solution in a two-phase mixed fluid supply channel (a second passage of the mixing component, described later). When cleaning the surgical instrument 10 with a cleaning agent-water solution or a cleaning agent- physiological saline solution, it provides an improved cleaning effect on fat and greasy dirt.

The mixing component is located in the conical portion 42 of the housing member. The mixing component includes a first passage 13 for introducing a second fluid; and a second passage 32 configured to extend from the proximal end of the housing member to the distal end of the housing member along a conical surface of the conical portion 42, and configured for mixing and delivering the first fluid and the second fluid. In another embodiment, the mixing component further includes a third passage 43. The first passage 13 and the third passage 43 are arranged at an angle α, which is less than or equal to 90 degrees, in order to ensure that the liquid received from the first fluid supply channel 31 and input into the third passage 43 and the gas output from the first passage 13 can be well mixed in the second passage 32, and the gas will not enter the third passage 43. **In** the second passage 32, especially the gas from the first passage 13 merges with especially the liquid from the first fluid supply channel 31, and a uniform fluid mixture, especially a gas-liquid mixture is formed here, which then passes through the third supply channel section 33. **In** an embodiment, the gas and/or liquid may be adjusted to have a temperature similar to that of the usage environment (for example, the body cavity of a patient) where the distal end part of the surgical instrument 10 is located, so as to avoid condensation and fogging. As used herein, a "uniform" two-phase fluid mixture, such as the two-phase fluid mixture in this embodiment, does not mean an absolutely average distribution of the gas phase and the liquid phase, but a substantially uniform distribution of both. For example, a gas-liquid two-phase fluid mixture can be regarded as "uniform" when there is no one or more long sections completely occupied by gas or liquid in its flow channel. When the surgical instrument 10 is cleaned with gas-liquid two-phase fluid, since the liquid component in the fluid is presented as discontinuous pulses, the gas component immediately following the liquid component will provide the liquid component with a thrust force towards the downstream of the fluid channel due to the faster speed of the gas component in the two-phase mixed fluid supply channel (i.e., the second passage 32 of the mixing component), which causes the fluid jets to be ejected onto the distal end part 18 of the surgical instrument 10 stronger, thereby enhancing the washing intensity of the cleaning fluid on the distal end part 18 of the surgical instrument 10 and obtaining a better cleaning effect.

**In** another embodiment, the first passage 13 is provided with a check valve 38 for blocking the first fluid from the third passage 43, and the check valve is selected from one of a duckbill valve, a membrane check valve and a spring-loaded ball check valve, preferably a duckbill valve.

**In** another embodiment, the first passage 13 has a switching device (not shown), such as a switching valve and a manifold, in order to switch between gas and cleaning agent without the need of removing the gas source from the inlet and reconnecting the cleaning agent source, and vice versa.

For example, during a surgical procedure assisted by the surgical instrument 10, for example, a vacuum pump, a vacuum source used as a hospital infrastructure, and conventional gas source (e.g., a CO₂ gas cylinder, optionally with a pressure reducing valve to adjust the pressure) and liquid source (e.g., a physiological saline bottle/bag, a peristaltic pump, a syringe, etc.) in a hospital are commonly used. According to the range of working pressure of the above-mentioned commonly used vacuum source, gas source and liquid source, the magnitude of the cross-sectional area at the outlet 23 of the first channel section 11 with varied cross-sectional area of flow, the magnitude of the cross-sectional area of the first passage 13 and the magnitude of the cross-sectional area of the third passage 32 can be appropriately selected, so as to achieve the best mixing effect of liquid with gas to obtain a uniform fluid mixture, including a gas-liquid two-phase fluid mixture and a cleaning agent-physiological saline mixture, etc., by way of example.

In this embodiment, the working process of cleaning the surgical instrument through the access assembly is as follows: when the surgical instrument is withdrawn to the interior of the access assembly for cleaning, the fluid supply channel is opened, and the check valve on the first passage is opened under the action of negative pressure, so that the second fluid flows into the access assembly to be mixed with the first fluid, which flows into the access assembly at the same time, to form a two-phase flow composed of the first fluid and the second fluid, for cleaning the distal end part of the surgical instrument. After the completion of cleaning, the surgical instrument enters the abdominal cavity of the patient, the fluid supply channel is closed, and the check valve automatically closes to prevent the first fluid from flowing out of the access assembly 1.

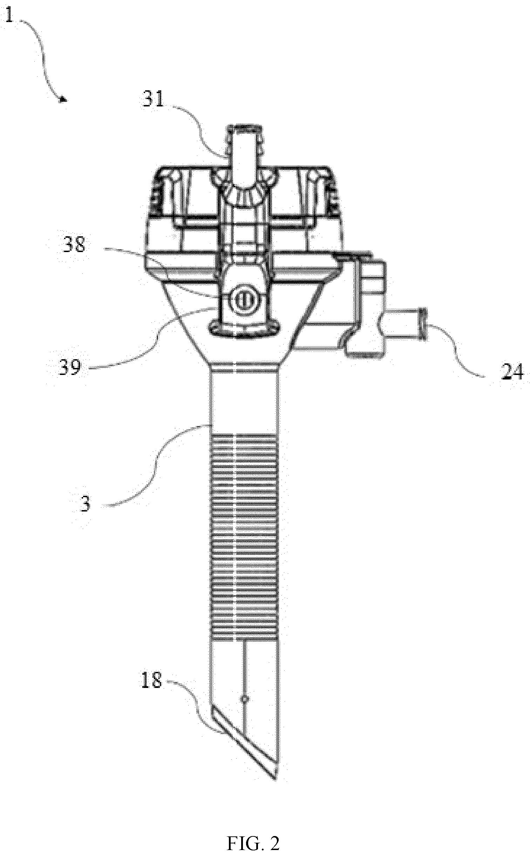

FIG. 2 is a side view of a non-limiting embodiment of the access assembly in Fig. 1. In a non-limiting embodiment, the first passage 13 may extend in a plane defined by the longitudinal axis ZZ' of the tube member 3 and the first fluid supply channel 31.

The access assembly 1 is further provided with an optional gas inlet 24 connected to an optional external gas source and configured to form an artificial pneumoperitoneum, a gas discharge port (not labeled) configured to discharge the gas from the gas source into the body cavity of the patient, and a gas flow channel (not labeled) configured to connect the gas inlet 24 with the gas discharge port. **In** some embodiments, the gas discharge port is located outside the distal end of the tube member 3 so that the gas received from the gas source can be discharged into the body cavity of the patient without passing through a lumen. **In** some embodiments, the arrangement of the optional gas inlet 24 of the access assembly 1 is similar to that of a conventional surgical instrument 10, such as a puncture device, which injects gas, such as CO₂, into the body cavity through an incision on the patient's body under a positive pressure to assist in forming a space occupied by gas, such as an artificial pneumoperitoneum, thereby providing a visual field and an operative space for observation and surgical operation of the surgical instrument 10. Advantageously, by discharging gas from the outside of the distal end of the tube member 3 without passing through the lumen, it can prevent the gas for forming the artificial pneumoperitoneum from being affected by the liquid and dirt in the lumen during the cleaning of the distal end part 18.

As described above, the access assembly provided by embodiments of the present disclosure includes: a housing member arranged at a proximal end of the access assembly, the housing member including a longitudinal channel for accessing the surgical instrument; a tube member arranged at a distal end of the access assembly, extending along a longitudinal axis of the access assembly, and configured to receive the surgical instrument; a proximal sealing device, arranged inside the housing member, and configured to connect a proximal end of the housing member with the received surgical instrument in a sealed manner; a distal sealing device connected with a distal end of the tube member in a sealed manner; and a fluid supply channel including a mixing component and a first fluid supply channel configured to input a first fluid. The first fluid supply channel is arranged at the proximal end of the housing member and extends in a direction from the proximal end of the housing member to a distal end of the housing member. The mixing component is arranged at the distal end of the housing member and is in fluid communication with the first fluid supply channel. The mixing component includes: a first passage configured to introduce a second fluid; and a second passage configured to extend from the distal end of the housing member to a proximal end of the tube member, and configured for mixing and delivering the first fluid and the second fluid. Through the above technical solution of the present disclosure, an access assembly with compact structure and simple production can be realized. The access assembly can not only automatically generate a two-phase flow but also improve the intensity of cleaning the surgical instrument, especially the lens of an endoscope, because when the endoscope is cleaned with a gas-liquid two-phase flow, the liquid component in the flow is presented as discontinuous pulses, and the gas component immediately following the liquid component will exert a thrust force towards the downstream of the fluid channel on the liquid component due to the faster speed of the gas component in the two-phase mixed fluid supply channel, which causes the fluid jets to be ejected onto the lens of the endoscope stronger. This enhances the washing intensity of the cleaning fluid on the lens of the endoscope and achieves better cleaning effect.

According to a developed solution of the present disclosure, it is advantageously provided that the mixing component further includes a third passage configured to be communicated with the first fluid supply channel. The third passage is configured to be matched with the conical-shaped structure of the housing member, and also be matched with the outlet of the first passage and the fluid outlet of the first fluid supply channel in their positions in a better way.

According to a developed solution of the present disclosure, it is advantageously provided that the housing member also includes a shell component surrounding an outer side of the longitudinal channel. The shell component at least includes a conical portion and a cylindrical portion connected with each other, the mixing component is arranged in the conical portion, and the first fluid supply channel is arranged in the cylindrical portion. Through this technical solution, it can be ensured that the channel of the second fluid is arranged downstream of the first fluid supply channel, and meanwhile facilitating the processing of the first fluid supply channel without enlarging the size of the transverse contour of the access assembly.

According to the present disclosure, the mixing component is arranged in the conical portion of the housing member, which is particularly beneficial for gas-liquid mixing in the access assembly and simplifies the processing technology.

According to a further developed solution of the present disclosure, it's provided that the third passage is arranged at an angle α relative to the first passage, and a value of the angle α is less than or equal to 90 degrees, and an extension direction of the first passage is from the proximal end of the housing member to the distal end of the housing member along a conical surface of the conical portion. This technical solution is particularly beneficial to inputting the second fluid, particularly preferably gas, with a viscosity significantly smaller than that of the first fluid, into the mixed fluid channel and causes the second fluid and the first fluid to form a sequence there in which the two types of fluids are alternately arranged to strengthen the washing intensity of the mixed fluid. The angle is smaller than or equal to 90 degrees, which ensures that the liquid output from the liquid supply channel and the gas output from the gas supply channel can be well mixed in the mixed fluid supply channel, and ensures that the gas will not enter the liquid supply channel.

Advantageously, according to the present disclosure, a check valve is arranged in the first passage, and the check valve is configured to block the first fluid from the first fluid supply channel from flowing into the first passage. Under the action of negative pressure, the check valve on the first passage opens, and the second fluid flows into the access assembly to be mixed with the first fluid, which flows in the access assembly at the same time, to form a mixed fluid flow, especially a gas-liquid two-phase flow, which cleans the end of the surgical instrument, especially the laparoscopic lens. After cleaning the surgical instrument, the surgical instrument enters the abdominal cavity again. Since the check valve only opens in a direction along which the second fluid enters, the first passage automatically closes in a direction opposite to the direction along which the fluid enters, so as to prevent the second fluid from flowing out of the access assembly.

According to the present disclosure, the check valve is designed as a duckbill valve, or the check valve is designed as a membrane check valve or a spring-loaded ball check valve, which is particularly beneficial to preventing the second fluid from flowing out of the access assembly.

According to a further developed solution of the present disclosure, the first fluid supply channel includes a second channel section, a first channel section and a third channel section which are sequentially communicated from a distal end to a proximal end of the first fluid supply channel; and a cross-sectional area of the proximal end of the first fluid supply channel is larger than a cross-sectional area of the distal end of the first fluid supply channel, so the resistance to the first fluid can be increased and the flow rate of the first fluid can be reduced, thereby facilitating the first fluid to be mixed with the second fluid.

The first channel section is configured such that its cross-sectional area of flow decreases in a stepwise manner in a direction of fluid flow, which is particularly advantageous to reduce the velocity of the first fluid and to facilitate processing.

As an alternative or in addition to the above solution, the first channel section can also be configured such that its cross-sectional area of flow decreases continuously in the direction of fluid flow.

According to further provisions of the present disclosure, a cross-sectional area of the second channel section is constant; and/or, the cross-sectional area of the second channel section is equal to a cross-sectional area of the first channel section at a first outlet of the first channel section. Since the cross-sectional area of flow of the second channel section in the direction of fluid flow is unchanged and is equal to the cross-sectional area of flow of the first channel section at the outlet of the first channel section, the resistance to the first fluid is further increased and the flow rate of the first fluid is reduced, thereby improving the effect of the first fluid mixing with the second gas at the downstream.

According to a developed solution of the present disclosure, the first passage is also configured to introduce a third fluid, such as cleaning agent; and the first passage further has a switching device for switching between the second fluid and the third fluid, such as a switching valve and a manifold. The switching device enables switching between gas and cleaning agent without the need of removing a second fluid source, i.e., a gas source, from the port of the first passage and reconnecting a cleaning agent source.
It is particularly advantageous to enhance the washing intensity of the cleaning fluid by selecting the first fluid to have a significantly greater viscosity than that of the second fluid. It is particularly preferred to select the first fluid as liquid, such as physiological saline, etc., and to select the second fluid as gas, such as carbon dioxide gas, etc.

## Claims

1. An access assembly (1) for a surgical instrument (10), comprising:
a housing member (40), arranged at a proximal end of the access assembly (1), the housing member (40) comprising a longitudinal channel for accessing the surgical instrument (10);
a tube member (3), arranged at a distal end of the access assembly (1), structured to extend along a longitudinal axis of the access assembly (1), and configured to receive the surgical instrument (10);
a proximal sealing device (25), arranged inside the housing member (40) and configured to connect a proximal end of the housing member (40) and the received surgical instrument (10) in a sealed manner;
a distal sealing device (2), connected with a distal end of the tube member (3) in a sealed manner; and
a fluid supply channel, comprising a mixing component and a first fluid supply channel (31) configured to input a first fluid, wherein the first fluid supply channel (31) is arranged at the proximal end of the housing member (40) and extends in a direction from the proximal end of the housing member (40) to a distal end of the housing member (40), wherein the mixing component comprises:
a first passage (13), configured to introduce a second fluid; and
a second passage (32),
**characterized by** that
the mixing component is arranged at the distal end of the housing member (40) and is in fluid communication with the first fluid supply channel (31);
wherein the second passage (32) is configured to extend from the distal end of the housing member (40) to a proximal end of the tube member (3), and is configured for mixing and delivering the first fluid and the second fluid.

2. The access assembly (1) according to claim 1, wherein the mixing component further comprises a third passage (43), and the third passage (43) is communicated with the first fluid supply channel (31).

3. The access assembly (1) according to claim 1, wherein the housing member (40) further comprises a shell component (4) surrounding an outer side of the longitudinal channel, the shell component (4) at least comprises a conical portion (42) and a cylindrical portion (41) connected with each other,
the mixing component is arranged in the conical portion (42), and the first fluid supply channel (31) is arranged in the cylindrical portion (41).

4. The access assembly (1) according to claim 3, wherein an extension direction of the second passage (32) is a direction from the proximal end of the housing member (40) to the distal end of the housing member (40) along a conical surface of the conical portion (42).

5. The access assembly (1) according to claim 2, wherein the third passage (43) is arranged at an angle α relative to the first passage (13), and a value of the angle α is less than or equal to 90 degrees.

6. The access assembly (1) according to claim 1, wherein a check valve (38) is arranged in the first passage (13), and the check valve (38) is configured to prevent the first fluid from the first fluid supply channel (31) from flowing into the first passage (13).

7. The access assembly (1) according to claim 6, wherein the check valve (38) is one selected from the group consisted of a duckbill valve, a membrane check valve and a spring-loaded ball check valve.

8. The access assembly (1) according to claim 1, wherein the first fluid supply channel (31) comprises a second channel section (12), a first channel section (11) and a third channel section (15) which are sequentially communicated from a distal end to a proximal end of the first fluid supply channel (31), and
a cross-sectional area of the proximal end of the first fluid supply channel (31) is larger than that of the distal end of the first fluid supply channel (31).

9. The access assembly (1) according to claim 8, wherein a cross-sectional area of the first channel section (11) decreases in a stepwise manner from the proximal end to the distal end of the access assembly (1); or, the cross-sectional area of the first channel section (11) continuously decreases in a direction from the proximal end to the distal end of the access assembly (1).

10. The access assembly (1) according to claim 8, wherein a cross-sectional area of the second channel section (12) is constant.

11. The access assembly (1) according to claim 8, wherein a cross-sectional area of the first passage (13) is greater than a cross-sectional area of the second channel section (12).

12. The access assembly (1) according to claim 1, wherein the first passage (13) is further configured to introduce a third fluid, and the first passage (13) is further provided with a switching device configured to switch between the second fluid and the third fluid.

13. The access assembly (1) according to claim 1, wherein the first fluid is liquid and the second fluid is gas.

## Patentansprüche

1. Zugangsvorrichtung (1) für ein chirurgisches Instrument (10), aufweisend:
ein Gehäuseelement (40), das an einem proximalen Ende der Zugangsvorrichtung (1) angeordnet ist, wobei das Gehäuseelement (40) einen Längskanal für einen Zugang zum chirurgischen Instrument (10) aufweist,
ein Rohrelement (3), das an einem distalen Ende der Zugangsvorrichtung (1) angeordnet ist, strukturiert ist, um sich entlang einer Längsachse der Zugangsvorrichtung (1) zu erstrecken, und zum Aufnehmen des chirurgischen Instruments (10) konfiguriert ist,
eine proximale Dichtungsvorrichtung (25), die innerhalb des Gehäuseelements (40) angeordnet ist und konfiguriert ist, um ein proximales Ende des Gehäuseelements (40) und das aufgenommene chirurgische Instrument (10) auf eine dichte Weise zu verbinden,
eine distale Dichtungsvorrichtung (2), die mit einem distalen Ende des Rohrelements (3) auf eine dichte Weise verbunden ist, und
einen Fluidzuführkanal, der eine Mischkomponente und einen Erstes-Fluid-Zuführkanal (31) aufweist, der konfiguriert ist, um ein erstes Fluid einzugeben, wobei der Erstes-Fluid-Zuführkanal (31) am proximalen Ende des Gehäuseelements (40) angeordnet ist und sich in einer Richtung von dem proximalen Ende des Gehäuseelements (40) zu einem distalen Ende des Gehäuseelements (40) erstreckt, wobei die Mischkomponente aufweist:
einen ersten Durchgang (13), der zum Einleiten eines zweiten Fluids konfiguriert ist, und
einen zweiten Durchgang (32),
**dadurch gekennzeichnet, dass**
die Mischkomponente am distalen Ende des Gehäuseelements (40) angeordnet ist und in Fluidkommunikation mit dem Erstes-Fluid-Zuführkanal (31) ist,
wobei der zweite Durchgang (32) konfiguriert ist, um sich vom distalen Ende des Gehäuseelements (40) zu einem proximalen Ende des Rohrelements (3) zu erstrecken, und zum Mischen und Abgeben des ersten Fluids und des zweiten Fluids konfiguriert ist.

2. Zugangsvorrichtung (1) gemäß Anspruch 1, wobei die Mischkomponente ferner einen dritten Durchgang (43) aufweist und der dritte Durchgang (43) mit dem Erstes-Fluid-Zuführkanal (31) in Verbindung ist.

3. Zugangsvorrichtung (1) gemäß Anspruch 1, wobei das Gehäuseelement (40) ferner eine Hüllenkomponente (4) aufweist, die eine Außenseite des Längskanals umgibt, wobei die Hüllenkomponente (4) mindestens einen konischen Abschnitt (42) und einen zylindrischen Abschnitt (41) aufweist, die miteinander verbunden sind,
wobei die Mischkomponente in dem konischen Abschnitt (42) angeordnet ist und der Erstes-Fluid-Zuführkanal (31) in dem zylindrischen Abschnitt (41) angeordnet ist.

4. Zugangsvorrichtung (1) gemäß Anspruch 3, wobei eine Erstreckungsrichtung des zweiten Durchgangs (32) eine Richtung vom proximalen Ende des Gehäuseelements (40) zum distalen Ende des Gehäuseelements (40) entlang einer konischen Fläche des konischen Abschnitts (42) ist.

5. Zugangsvorrichtung (1) gemäß Anspruch 2, wobei der dritte Durchgang (43) in einem Winkel α relativ zum ersten Durchgang (13) angeordnet ist und ein Wert des Winkels α kleiner oder gleich 90 Grad ist.

6. Zugangsvorrichtung (1) gemäß Anspruch 1, wobei ein Rückschlagventil (38) im ersten Durchgang (13) angeordnet ist und das Rückschlagventil (38) konfiguriert ist, um zu verhindern, dass das erste Fluid aus dem Erstes-Fluid-Zuführkanal (31) in den ersten Durchgang (13) fließt.

7. Zugangsvorrichtung (1) gemäß Anspruch 6, wobei das Rückschlagventil (38) eines ist, das aus der Gruppe ausgewählt ist, die aus einem Entenschnabelventil, einem Membranrückschlagventil und einem federbelasteten Kugelrückschlagventil besteht.

8. Zugangsvorrichtung (1) gemäß Anspruch 1, wobei der Erstes-Fluid-Zuführkanal (31) einen zweiten Kanalabschnitt (12), einen ersten Kanalabschnitt (11) und einen dritten Kanalabschnitt (15) aufweist, die nacheinander von einem distalen Ende zu einem proximalen Ende des Erstes-Fluid-Zuführkanals (31) in Verbindung sind, und
wobei eine Querschnittsfläche des proximalen Endes des Erstes-Fluid-Zuführkanals (31) größer als die des distalen Endes des Erstes-Fluid-Zuführkanals (31) ist.

9. Zugangsvorrichtung (1) gemäß Anspruch 8, wobei eine Querschnittsfläche des ersten Kanalabschnitts (11) vom proximalen Ende zum distalen Ende der Zugangsvorrichtung (1) schrittweise abnimmt, oder wobei die Querschnittsfläche des ersten Kanalabschnitts (11) in einer Richtung vom proximalen Ende zum distalen Ende der Zugangsvorrichtung (1) kontinuierlich abnimmt.

10. Zugangsvorrichtung (1) gemäß Anspruch 8, wobei eine Querschnittsfläche des zweiten Kanalabschnitts (12) konstant ist.

11. Zugangsvorrichtung (1) gemäß Anspruch 8, wobei eine Querschnittsfläche des ersten Durchgangs (13) größer als eine Querschnittsfläche des zweiten Kanalabschnitts (12) ist.

12. Zugangsvorrichtung (1) gemäß Anspruch 1, wobei der erste Durchgang (13) ferner zum Einleiten eines dritten Fluids konfiguriert ist und der erste Durchgang (13) ferner mit einer Schaltvorrichtung versehen ist, die zum Umschalten zwischen dem zweiten Fluid und dem dritten Fluid konfiguriert ist.

13. Zugangsvorrichtung (1) gemäß Anspruch 1, wobei das erste Fluid flüssig ist und das zweite Fluid Gas ist.

## Revendications

1. Ensemble d'accès (1) pour un instrument chirurgical (10), comprenant :
un élément de boîtier (40), disposé à une extrémité proximale de l'ensemble d'accès (1), l'élément de boîtier (40) comprenant un canal longitudinal pour accéder à l'instrument chirurgical (10) ;
un élément de tube (3), disposé à une extrémité distale de l'ensemble d'accès (1), structuré pour s'étendre le long d'un axe longitudinal de l'ensemble d'accès (1), et configuré pour recevoir l'instrument chirurgical (10) ;
un dispositif d'étanchéité proximal (25), disposé à l'intérieur de l'élément de boîtier (40) et configuré pour relier de manière étanche une extrémité proximale de l'élément de boîtier (40) et l'instrument chirurgical (10) reçu ;
un dispositif d'étanchéité distal (2), relié de manière étanche à une extrémité distale de l'élément de tube (3) ; et
un canal d'alimentation en fluide, comprenant un composant de mélange et un canal d'alimentation en premier fluide (31) configuré pour introduire un premier fluide, dans lequel le canal d'alimentation en premier fluide (31) est disposé à l'extrémité proximale de l'élément de boîtier (40) et s'étend dans une direction allant de l'extrémité proximale de l'élément de boîtier (40) à une extrémité distale de l'élément de boîtier (40), dans lequel le composant de mélange comprend :
un premier passage (13), configuré pour introduire un deuxième fluide ; et
un deuxième passage (32),
**caractérisé en ce que**
le composant de mélange est disposé à l'extrémité distale de l'élément de boîtier (40) et est en communication fluidique avec le canal d'alimentation en premier fluide (31) ;
dans lequel le deuxième passage (32) est configuré pour s'étendre depuis l'extrémité distale de l'élément de boîtier (40) jusqu'à une extrémité proximale de l'élément de tube (3), et est configuré pour mélanger et délivrer le premier fluide et le deuxième fluide.

2. Ensemble d'accès (1) selon la revendication 1, dans lequel le composant de mélange comprend en outre un troisième passage (43), et le troisième passage (43) communique avec le canal d'alimentation en premier fluide (31).

3. Ensemble d'accès (1) selon la revendication 1, dans lequel l'élément de boîtier (40) comprend en outre un composant de coque (4) entourant un côté extérieur du canal longitudinal, le composant de coque (4) comprend au moins une partie conique (42) et une partie cylindrique (41) reliées l'une à l'autre,
le composant de mélange est disposé dans la partie conique (42), et le canal d'alimentation en premier fluide (31) est disposé dans la partie cylindrique (41).

4. Ensemble d'accès (1) selon la revendication 3, dans lequel une direction d'extension du deuxième passage (32) est une direction allant de l'extrémité proximale de l'élément de boîtier (40) à l'extrémité distale de l'élément de boîtier (40) le long d'une surface conique de la partie conique (42).

5. Ensemble d'accès (1) selon la revendication 2, dans lequel le troisième passage (43) est disposé selon un angle α par rapport au premier passage (13), et une valeur de l'angle α est inférieure ou égale à 90 degrés.

6. Ensemble d'accès (1) selon la revendication 1, dans lequel un clapet anti-retour (38) est disposé dans le premier passage (13), et le clapet anti-retour (38) est configuré pour empêcher le premier fluide provenant du canal d'alimentation en premier fluide (31) de s'écouler dans le premier passage (13).

7. Ensemble d'accès (1) selon la revendication 6, dans lequel le clapet anti-retour (38) est un qui est choisi dans le groupe constitué par un clapet à bec de canard, un clapet anti-retour à membrane et un clapet anti-retour à bille à ressort.

8. Ensemble d'accès (1) selon la revendication 1, dans lequel le premier canal d'alimentation en fluide (31) comprend une deuxième section de canal (12), une première section de canal (11) et une troisième section de canal (15) qui sont reliées séquentiellement d'une extrémité distale à une extrémité proximale du canal d'alimentation en premier fluide (31), et
une surface de section transversale de l'extrémité proximale du canal d'alimentation en premier fluide (31) est plus grande que celle de l'extrémité distale du canal d'alimentation en premier fluide (31).

9. Ensemble d'accès (1) selon la revendication 8, dans lequel une surface de section transversale de la première section de canal (11) diminue de manière progressive de l'extrémité proximale à l'extrémité distale de l'ensemble d'accès (1) ; ou la surface de section transversale de la première section de canal (11) diminue de manière continue dans une direction allant de l'extrémité proximale à l'extrémité distale de l'ensemble d'accès (1).

10. Ensemble d'accès (1) selon la revendication 8, dans lequel une surface de section transversale de la deuxième section de canal (12) est constante.

11. Ensemble d'accès (1) selon la revendication 8, dans lequel une surface de section transversale du premier passage (13) est supérieure à une surface de section transversale de la deuxième section de canal (12).

12. Ensemble d'accès (1) selon la revendication 1, dans lequel le premier passage (13) est en outre configuré pour introduire un troisième fluide, et le premier passage (13) est en outre muni d'un dispositif de commutation configuré pour commuter entre le deuxième fluide et le troisième fluide.

13. Ensemble d'accès (1) selon la revendication 1, dans lequel le premier fluide est un liquide et le deuxième fluide est un gaz.
